(19) **Europäisches Patentamt European Patent Office Office européen des brevets**

(11) **EP 4 678 758 A1**

(12) **EUROPEAN PATENT APPLICATION**
published in accordance with Art. 153(4) EPC

(43) Date of publication:
14.01.2026 Bulletin 2026/03

(21) Application number: 24771246.6

(22) Date of filing: 14.03.2024

(51) International Patent Classification (IPC):
*C12P 13/00* (2006.01)   *C12N 1/20* (2026.01)
*B01D 61/00* (2006.01)   *B01D 17/00* (2006.01)

(52) Cooperative Patent Classification (CPC):
B01D 17/00; B01D 61/00; C12N 1/20; C12P 13/00

(86) International application number:
PCT/KR2024/095489

(87) International publication number:
WO 2024/191247 (19.09.2024 Gazette 2024/38)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 14.03.2023 KR 20230033333

(71) Applicant: CJ Cheiljedang Corporation
Seoul 04560 (KR)

(72) Inventors:
• SHIN, Ji Hyun
Seoul 04560 (KR)

• KANG, Ji-hun
Seoul 04560 (KR)
• PARK, Sang Min
Seoul 04560 (KR)
• CHOI, Hoon Seob
Seoul 04560 (KR)
• KWON, Min Kyung
Seoul 04560 (KR)
• KIM, Min Sup
Seoul 04560 (KR)

(74) Representative: Meissner Bolte Partnerschaft
mbB
Patentanwälte Rechtsanwälte
Postfach 86 06 24
81633 München (DE)

(54) **AMINO ACID PRODUCTION METHOD HAVING IMPROVED FILTRATION FLOW RATE**

(57) The present disclosure relates to a method for preparing an amino acid, comprising adding a coagulant and/or a flocculant to a culture broth comprising the amino acid.

EP 4 678 758 A1

## Description

**[Technical Field]**

The present disclosure relates to a method for preparing an amino acid with an improved filtration flux, comprising adding a coagulant and/or a flocculant to a culture broth comprising the amino acid.

**[Background Art]**

Amino acids are the fundamental building blocks of proteins and are used as important components of pharmaceutical raw materials, food additives, animal feed, nutritional supplements, insecticides, and fungicides. In particular, branched-chain amino acids (BCAAs), which collectively refer to the essential amino acids valine, leucine, and isoleucine, are known to have antioxidant effects and to directly promote protein synthesis in muscle cells.

Meanwhile, BCAA production using microorganisms is mainly achieved through microorganisms of the genus *Escherichia* or the genus *Corynebacterium,* and is known to biosynthesize BCAAs from pyruvic acid through multiple steps using 2-ketoisocaproic acid as a precursor. However, BCAA production using such microorganisms has a problem in that large-scale industrial manufacturing cannot be easily achieved.

Meanwhile, a general process for preparing amino acids is achieved by including a series of steps such as i) culturing microorganisms, ii) filtering the culture broth containing amino acids, iii) decolorization, iv) filtration, v) concentration, and vi) crystallization, wherein the unit processes may be added or omitted depending on the product characteristics. Among the above processes, the filtration process (filter process) is a process of removing cells, proteins, colloidal substances, natural organic matter (NOM), and low-molecular-weight substances contained in the culture broth using a filtration membrane. When the culture broth is filtered through the membrane in the filtration process, impurities deposit on the membrane over time, and a gel-like cake layer is formed on the membrane surface. Such membrane fouling reduces the membrane permeate flux. When the membrane permeate flux decreases, the daily product yield is reduced, the cleaning cycle of the filtration membrane becomes longer, and the membrane replacement interval becomes shorter, which increases maintenance costs and unit production costs, thereby reducing price competitiveness. Accordingly, there is a need for a method capable of removing impurities in the culture broth or reducing membrane fouling caused by impurities in the filtration process in order to suppress the formation of the cake layer by impurities on the membrane surface, which causes a decrease in membrane permeate flux.

**[Disclosure]**

**[Technical Problem]**

One object of the present disclosure is to provide a method for preparing an amino acid from a culture broth comprising an amino acid, comprising: (a) adding an organic coagulant, a cationic polymer flocculant, or both to the culture broth comprising an amino acid to precipitate impurities; and (b) subjecting the culture broth with precipitated impurities to membrane filtration.

Another object of the present disclosure is to provide a feed composition comprising an amino acid prepared by the method of the present disclosure.

**[Technical Solution]**

The present disclosure will be described in detail as follows. Meanwhile, each description and embodiment described herein can be applied to other descriptions and embodiments, respectively. That is, all combinations of various elements described herein fall within the scope of the present disclosure. Further, the scope of the present disclosure is not limited by the specific description described below.

Additionally, a number of papers and patent documents have been cited throughout the present specification. The content of the cited papers and patent documents is incorporated herein by reference in their entirety to describe the level of the technical field to which the present disclosure belongs and the contents of the present disclosure more clearly.

To achieve the above objects, one aspect of the present disclosure provides a method for preparing an amino acid from a culture broth comprising an amino acid, comprising: (a) adding an organic coagulant, a cationic polymer flocculant, or both to the culture broth comprising an amino acid to precipitate impurities; and (b) subjecting the culture broth with precipitated impurities to membrane filtration.

As used herein, the term "coagulant" may refer to a substance that curdles or clots particles in a liquid for separation from the liquid. Specifically, it refers to a substance that promotes the aggregation of fine particles in a liquid into larger masses. For example, since particles in a liquid carry surface charges that repel each other, they do not precipitate but remain suspended in water. A coagulant imparts opposite charges to these particles, destabilizing the charges and thereby causing the particles to cling to each other. The coagulant is applied across various industrial fields ranging from food, beverages, chemicals, and pharmaceuticals to drinking water, municipal wastewater treatment, and oil and gas drilling. It can be used to precipitate relatively small particles, typically several $\mu$m in size, for example, about 2 $\mu$m.

As used herein, the term "flocculant", "flocculating agent", or "flocking agent" refers to a substance used to remove suspended solids from a liquid by inducing flocculation whereby solids aggregate to form flakes that settle at the bottom, and is also called a clarifying agent. The larger and/or heavier particles formed therein are also referred to as flocs. Unlike coagulants, which induce chemical flocculation by charge destabilization, flocculants promote physical flocculation by flocculating colloids and other suspended particles in a liquid and thereby forming flocs. Such flocculants may be used in filtration processes, such as water-treatment processes, to precipitate small particles and improve filterability. They can typically be used to precipitate relatively large particles having a size of 10 $\mu$m or more.

For example, the coagulant or flocculant used in the method of the present disclosure may be an organic coagulant, a cationic polymer flocculant, or a combination thereof.

Specifically, the organic coagulant may include poly(dimethylamine-co-epichlorohydrin) (EPI-AMINE) or poly(diallyldimethylammonium chloride) (polyDADMAC), and the cationic polymer flocculant may include long chain polymer components such as polyacrylamide (PAM), polyethylenimine (PEI), or polyvinylamine (PVAM); however, the present disclosure is not limited thereto.

For example, the production method of the present disclosure may further comprise adjusting the culture broth comprising an amino acid to an acidic pH of 3 to 5. For example, the culture broth comprising an amino acid may be adjusted to pH 3.0 to 4.5, pH 3.5 to 4.0, pH 3.7 to 4.3, or pH 3.8 to 4.2, but is not limited thereto. The specific embodiments of the present disclosure confirm that the flocculation efficiency of a coagulant or flocculant is affected by the pH conditions of the solution, and that, in particular, improved flocculation efficiency is exhibited under acidic conditions compared to neutral conditions. In addition to pH conditions, the flocculation efficiency of a coagulant or flocculant is also affected by the concentration of the coagulant or flocculant, the type of amino acid to be prepared, the type, size, and/or amount of impurities in the culture broth, *etc.* Thus, the scope of the present disclosure is not limited to such pH conditions.

The method for producing an amino acid of the present disclosure is characterized in that the filtration flux of membrane filtration is improved compared to that without treatment with a coagulant and/or flocculant.

Furthermore, the method for producing an amino acid of the present disclosure can achieve the effect of improving the chromaticity of a membrane-filtered filtrate and reducing the content of impurities in the filtrate.

For example, the culture broth comprising an amino acid may comprise, as impurities, one or more selected from the group consisting of cells, proteins, colloidal substances, natural organic matter (NOM), and low-molecular-weight substances. A culture broth for producing amino acids may comprise various impurities including colloidal substances derived from fermentation by-products such as fermentation media and cell lysates in addition to suspension materials such as cells. These particles can have adverse effects on productivity by adhering to the membrane in the subsequent filtration process of the amino-acid production process and thereby forming a cake layer, which not only reduces filtration efficiency but also contaminates the membrane and shortens the membrane replacement interval. Meanwhile, the use of a coagulant and/or flocculant in the method of the present disclosure aggregates/coagulates these impurities, leading to precipitation and thereby markedly reducing fouling such as cake layer formation on the membrane. Thus, it can achieve the effects of improving the membrane permeate flux and prolonging the membrane lifetime, which enhance not only productivity but also the economics of production.

In the present disclosure, the culture broth comprising an amino acid may be obtained through fermentation by a microorganism, but is not limited thereto. In particular, the fermentation by a microorganism may be performed using methods known in the art without limitation.

For example, the production method of the present disclosure may further comprise a pre-treatment step of separating the cells from the culture broth comprising an amino acid. For example, the cell separation may be performed using a mechanical separator (MS) or a membrane filter (MF), but is not limited thereto. Cell separation using a mechanical separator typically employs centrifugation, but in such cases, colloidal substances may not be separated and may pass into the filtrate. Therefore, when combined with the production method of the present disclosure, it can exhibit a particularly remarkable improvement effect.

As used herein, the term "culture broth" refers to a culture product obtained by culturing a microorganism. The culture broth may comprise the cultured microorganism. Moreover, the culture broth may be used interchangeably with "fermentation broth".

As used herein, the term "culture broth comprising an amino acid" may be used interchangeably with "amino acid-containing culture broth" or "amino acid culture broth".

In the present disclosure, the culture broth comprising an amino acid may be a fermentation product comprising cells obtained by a known microorganism fermentation method, a liquid obtained by removing cells from the fermentation product, or a concentrate obtained by concentrating the same (US 8465962 B2, US 9885093 B2, US 10351859 B2, US 7863435 B2, US 10787692 B2, US 9029105 B2, US 2021-0094903 A1), but is not limited thereto.

Specifically, the culture broth comprising an amino acid of the present disclosure may be obtained by culturing or fermenting a microorganism that produces the corresponding amino acid, and the microorganism and a method of culturing or fermenting the same can be selected and used by those skilled in the art from known types and methods. For example, the microorganism includes both wild-type microorganisms and microorganisms that have undergone natural or artificial genetic modification. It may be a microorganism (US 9587261 B2, US 7863435 B2, *etc.*) that has undergone genetic modification or has enhanced activity for the production of a target L-amino acid, in which a specific mechanism is weakened or enhanced due to factors such as the insertion of foreign genes or the enhancement or inactivation of endogenous gene activity. Specifically, while the microorganism is not particularly limited in its type as long as it can produce the desired amino acid, it may be a microorganism of the genera *Enterobacter, Escherichia, Erwinia, Serratia, Providencia, Corynebacterium,* and *Brevibacterium.* More specifically, it may be a microorganism of the genus *Corynebacterium* or *Escherichia.* The microorganism of the genus Corynebacterium may be *Corynebacterium glutamicum, Corynebacterium ammoniagenes, Corynebacterium thermoaminogenes, Corynebacterium efficiens, Corynebacterium stationis, Corynebacterium phocae, Corynebacterium flavescens, Corynebacterium humireducens, Corynebacterium halotolerans, Corynebacterium pollutisoli, Corynebacterium marinum, Corynebacterium freiburgense, Corynebacterium cystitidis, Corynebacterium durum, Corynebacterium pilosum,* or *Corynebacterium testudinoris,* but is not limited thereto. The microorganism of the genus *Escherichia* may be *Escherichia coli,* but is not limited thereto.

Additionally, the method of the present disclosure may further comprise, after the membrane filtration step, optionally one or more steps selected from the group consisting of a decolorization step, a filtrate concentration step, a crystallization step, a crystal separation step, a drying step, a separation step, and a product commercialization step. Each step can be performed using methods known in the art without limitation (for example, US 2021-0094903 A1). The specific conditions may be appropriately changed to optimize the process, but are not limited thereto.

For example, the amino acid that can be produced by the method of the present disclosure may be isoleucine, valine, or leucine, but is not limited thereto. Depending on the type of amino acid to be produced, the type and/or size of impurities contained in the culture broth may vary. Accordingly, with respect to the amino acid to be produced, a coagulant or flocculant suitable for improving the membrane permeate flux may be selected and used in consideration of the above factors, or both may be used in combination.

Another aspect of the present disclosure provides a feed composition comprising an amino acid prepared by the method of the present disclosure.

The amino acid of the present disclosure may be suitable for use as a feed additive in animal feed preparation. For example, as the feed additive, the amino acid may be mixed with feed materials by itself as part of an animal feed premix or as a precursor of animal feed. The feed composition comprising the amino acid may be administered to an animal either alone or in combination with other feed additives in edible carriers. Additionally, the feed composition may be readily administered to an animal as top dressing, by directly mixing it into the animal feed, or in a separate oral formulation.

## [Advantageous Effects]

The method of the present disclosure can not only improve productivity by precipitating impurities in the culture broth through the addition of a coagulant and/or flocculant to the culture broth, thereby reducing membrane fouling in the subsequent filtration process and improving membrane permeate flux, but also achieve improvement in the quality of the produced product by reducing the content of proteins and coloring substances in the filtrate.

## [Mode for Carrying Out the Invention]

Hereinafter, the present disclosure is explained in more detail by the following examples. However, these examples are set forth to illustrate the present disclosure, and the scope of the present disclosure is not limited thereto.

### Example 1: Effect of Addition of Coagulant/Flocculant in Flocculation Experiment

An amino acid culture broth produced using a microorganism was used as a process liquid. As the amino acid culture broth, isoleucine culture broth, valine culture broth, and leucine culture broth were provided. To test membrane filtration efficiency, numerous cells with large particle size were first removed by centrifugation using a mechanical separator (Alfa Laval Separator), and the supernatant was used as the feed for membrane filtration testing. The pH of the feed was 6, and the pH was adjusted using 98% $H_2SO_4$ and 50% NaOH.

Specifically, in the membrane filtration testing, 200 mL of the separation solution of the aforementioned amino acid culture broth from which cells had been primarily removed was prepared, and the pH was adjusted using 98% $H_2SO_4$ and 50% NaOH. To each of the solutions with adjusted pH, 200 ppm of each of the seven coagulants/flocculants listed in Table 1 was added, followed by rapid mixing for 10 seconds, slow mixing for 30 seconds, and settling for 30 minutes. The settled liquid was filtered through a filter cloth having a diameter of 3.6 cm and a membrane area of 0.001 $m^2$ fixed on a

vacuum filtration apparatus under reduced pressure. The process liquid was separated into precipitated agglomerate solids (cake) and a liquid filtrate using the filter cloth. The average membrane flux was measured based on the time taken for 200 mL of the filtrate to be discharged, and was calculated as the flow rate (L) per unit membrane area (m$^2$) per unit time (hr):

$$\text{Average membrane flux } (LMH) = \frac{\text{Filtrate volume}(0.2L)}{\text{Membrane area}(0.001\ m^2) \times \text{Measured discharge time }(hr)}$$

.

[Table 1]

| Product name | Component | Chemical name: | CAS No. | Active ingredient content (%) | Average molecular weight MW |
|---|---|---|---|---|---|
| BASF Magnafloc LT-7991 | EPI-AMINE | poly(dimethylamin e-co-epichlorohydrin) | 42751-79-1 | 50 (balance water) | 150,000 -300,000 |
| BASF Magnafloc LT-7994 | polyDADM AC | poly(diallyldimethy lammonium chloride) | 26062-79-3 | 40 (balance water) | 200,000 -350,000 |
| BASF Zetag8185 | PAM | polyacrylamide | 9003-05-08 | 88.75↑ | 10,000,000 |
| BASF Zetag IP 2M | PEI | polyethylenimine | 114133-44-7 | 24 (balance water) | 2,000,000 |
| BASF Hercobond™ 6960 | PVAM | polyvinylamine (formamide, ethenyl-, homopolymer, dimapa-quat conjugate, hydrochloride) | 1353435-58-1 | 10 (balance water) | 34,000 -1,500,000 |
| BIOSYNTH FP31706 | PAC | polyaluminum chloride | 1327-41-9 | min 99 | 100-1000 |
| Sigma-Aldrich 434957 | APAM | poly(acrylamide-co-acrylic acid) | 9003-06-9 | 87 | 15,000,000 |

**Example 2: Effect of pH in Flocculation Experiment**

To confirm the effect of pH in the above flocculation experiment, the experiment was carried out in the same manner as in Example 1, except that the pH was adjusted to 4 (acidic), 6 (neutral), and 8 (basic) and flocculation at each pH was examined. The results are shown in Tables 2 to 7 below. In brief, no precipitation was observed at pH 6 and pH 8 regardless of the type of coagulant/flocculant, whereas precipitation or no precipitation was observed at acidic pH 4 depending on the presence and/or type of coagulant/flocculant. Such results indicate that the combination of the addition of an appropriate coagulant/flocculant and use of acidic pH promotes precipitation and further improves filtration flux and filtrate quality.

**Example 3: Effect of Addition of Coagulant/Flocculant and pH in Isoleucine Production**

A membrane filtration experiment was carried out using, as the feed, the separation solution of the amino acid culture broth from which cells had been primarily removed as in Example 1, except that the amino acid culture broth used was a culture broth comprising isoleucine, the pH was adjusted to 4, 6, or 8, and the coagulants/flocculants in Table 1 were either included or excluded. The formation of precipitates in each sample was observed as in Example 1, and the permeate flux was calculated. The results are shown in Tables 2 and 3. Additionally, for each filtrate obtained, absorbance at 280 nm and 420 nm, which are used as indices of protein content and chromaticity, were measured. The results are shown together in Tables 2 and 3.

[Table 2]

| | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 |
|---|---|---|---|---|---|---|---|---|---|
| pH of process solution | 4 | 6 | 8 | 4 | 6 | 8 | 4 | 6 | 8 |
| Type of additive | Not added | | | PAC | | | APAM | | |
| **Results** | | | | | | | | | |
| Precipitate formation | X | X | X | O | X | X | X | X | X |
| Permeate flux (LMH) | 236 | 199 | 186 | 418 | 395 | 169 | 257 | 181 | 179 |
| UV (280nm) | 7.0 | 7.4 | 7.4 | 6.9 | 7.3 | 7.5 | 7.4 | 6.7 | 6.9 |
| UV (420nm) | 0.7 | 0.9 | 1.1 | 0.3 | 0.8 | 1.0 | 0.7 | 0.9 | 1.0 |

[Table 3]

| | Example 1 | Comparative Example 10 | Comparative Example 11 | Example 2 | Comparative Example 12 | Comparative Example 13 | Example 3 | Comparative Example 14 | Comparative Example 15 | Example 4 | Comparative Example 16 | Comparative Example 17 | Example 5 | Comparative Example 18 | Comparative Example 19 |
|---|---|---|---|---|---|---|---|---|---|---|---|---|---|---|---|
| pH of process solution | 4 | 6 | 8 | 4 | 6 | 8 | 4 | 6 | 8 | 4 | 6 | 8 | 4 | 6 | 8 |
| Type of additive | EPI-AMINE | | | polyDDMAC | | | PAM | | | PEI | | | PVAM | | |
| Results | | | | | | | | | | | | | | | |
| Precipitate formation | O | X | X | O | X | X | O | X | X | O | X | X | O | X | X |
| Permeate flux (LMH) | 1086 | 474 | 190 | 1276 | 398 | 171 | 1353 | 502 | 180 | 999 | 483 | 193 | 1032 | 431 | 188 |
| UV (280nm) | 5.4 | 7.0 | 7.3 | 5.2 | 7.1 | 7.3 | 5.7 | 7.2 | 7.7 | 5.8 | 7.1 | 6.9 | 5.2 | 6.9 | 7.3 |
| UV (420nm) | 0.1 | 0.8 | 0.9 | 0.1 | 0.8 | 0.9 | 0.1 | 0.8 | 0.9 | 0.1 | 0.8 | 1.2 | 0.1 | 0.8 | 1.2 |

As shown in Tables 2 and 3, in the samples without a coagulant/flocculant tested in the isoleucine production process, no precipitation was observed regardless of pH, and permeate flux was low. Among the groups treated with coagulants/flocculants, the group treated with APAM similarly showed no precipitation regardless of pH, and no improvement in permeate flux was observed compared to the control without a coagulant/flocculant. The group treated with the inorganic coagulant PAC showed precipitation at pH 4, but only a slight improvement in permeate flux was observed, and no precipitation was observed at the other pH values. Meanwhile, in the groups treated with EPI-AMINE, polyDADMAC, PAM, PEI, or PVAM, no precipitation and no improvement in permeate flux were observed in the test groups adjusted to pH 6 and 8, whereas precipitation and a significant increase in permeate flux were observed in the test groups adjusted to pH 4. In particular, the group treated with PAM was confirmed to have an improvement of about 5.7-fold in filtration flux. Moreover, these test groups exhibited a significant decrease in the absorbance at 420 nm, which is an index of chromaticity. Such results indicate that the quality of the filtrate can be improved by the above process.

**Example 4: Effect of Addition of Coagulant/Flocculant and pH in Valine Production**

A membrane filtration experiment was carried out using, as the feed, the separation solution of the amino acid culture broth from which cells had been primarily removed as in Example 1, except that the amino acid culture broth used was a culture broth comprising valine, the pH was adjusted to 4 or 6, and the coagulants/flocculants in Table 1 were either included or excluded. The formation of precipitates in each sample was observed as in Example 1, and the permeate flux was calculated. The results are shown in Tables 4 and 5. Additionally, for each filtrate obtained, absorbance at 280 nm and 420 nm, which are used as indices of protein content and chromaticity, were measured. The results are shown together in Tables 4 and 5.

[Table 4]

|  | Comparative Example 20 | Comparative Example 21 | Comparative Example 22 | Comparative Example 23 | Comparative Example 24 | Comparative Example 25 |
|---|---|---|---|---|---|---|
| pH of process solution | 4 | 6 | 4 | 6 | 4 | 6 |
| Type of additive | Not added | | PAC | | APAM | |
| Results | | | | | | |
| Precipitate formation | X | X | O | X | X | X |
| Permeate flux (LMH) | 1415 | 1074 | 1888 | 1172 | 1181 | 1036 |
| UV (280nm) | 19 | 21 | 19 | 22 | 19 | 19 |
| UV (420nm) | 2.4 | 2.8 | 1.8 | 3.0 | 3.2 | 2.6 |

[Table 5]

| | Example 6 | Comparative Example 26 | Example 7 | Comparative Example 27 | Example 8 | Comparate Example 28 | Example 9 | Comparative Example 29 | Example 10 | Comparative Example 30 |
|---|---|---|---|---|---|---|---|---|---|---|
| pH of process solution | 4 | 6 | 4 | 6 | 4 | 6 | 4 | 6 | 4 | 6 |
| Type of additive | EPI-AMINE | | polyDADMAC | | PAM | | PEI | | PVAM | |
| Results | | | | | | | | | | |
| Precipitate formation | O | X | O | X | O | X | O | X | O | X |
| Permeate flux (LMH) | 3931 | 1987 | 5661 | 1718 | 3022 | 1838 | 3825 | 1686 | 3629 | 1441 |
| UV (280nm) | 19 | 21 | 19 | 19 | 18 | 19 | 19 | 19 | 19 | 19 |
| UV (420nm) | 1.8 | 2.9 | 1.9 | 2.6 | 1.9 | 2.3 | 1.8 | 2.6 | 1.8 | 2.6 |

As shown in Tables 4 and 5, in the samples without a coagulant/flocculant tested in the valine production process, no precipitation was observed regardless of pH, and permeate flux was low. Among the groups treated with coagulants/-flocculants, the group treated with APAM similarly showed no precipitation regardless of pH, and no improvement in permeate flux was observed compared to the control without a coagulant/flocculant. The group treated with the inorganic coagulant PAC showed precipitation at pH 4, but only a slight improvement in permeate flux was observed, and no precipitation was observed at the other pH values. Meanwhile, in the groups treated with EPI-AMINE, polyDADMAC, PAM, PEI, or PVAM, no precipitation and no improvement in permeate flux were observed in the test groups adjusted to pH 6, whereas precipitation and a significant increase in permeate flux were observed in the test groups adjusted to pH 4. In particular, the group treated with polyDADMAC was confirmed to have an improvement of about 4.0-fold in filtration flux. Moreover, these test groups exhibited a significant decrease in the absorbance at 420 nm, which is an index of chromaticity. Such results indicate that the quality of the filtrate can be improved by the above process.

**Example 5: Effect of Addition of Coagulant/Flocculant and pH in Leucine Production**

A membrane filtration experiment was carried out using, as the feed, the separation solution of the amino acid culture broth from which cells had been primarily removed as in Example 1, except that the amino acid culture broth used was a culture broth comprising leucine, the pH was adjusted to 4 or 6, and the coagulants/flocculants in Table 1 were either included or excluded. The formation of precipitates in each sample was observed as in Example 1, and the permeate flux was calculated. The results are shown in Tables 6 and 7. Additionally, for each filtrate obtained, absorbance at 280 nm and 420 nm, which are used as indices of protein content and chromaticity, were measured. The results are shown together in Tables 6 and 7.

[Table 6]

| | Comparative Example 31 | Comparative Example 32 | Comparative Example 33 | Comparative Example 34 | Comparative Example 35 | Comparative Example 36 |
|---|---|---|---|---|---|---|
| pH of process solution | 4 | 6 | 4 | 6 | 4 | 6 |
| Type of additive | Not added | | PAC | | APAM | |
| Results | | | | | | |
| Precipitate formation | X | X | O | X | X | X |
| Permeate flux (LMH) | 693 | 561 | 847 | 636 | 707 | 578 |
| UV (280nm) | 19 | 20 | 10 | 24 | 17 | 19 |
| UV (420nm) | 3.2 | 4.3 | 2.0 | 4.7 | 3.0 | 3.6 |

[Table 7]

| | Example 11 | Comparative Example 37 | Example 12 | Comparative Example 38 | Example 13 | Comparative Example 39 | Example 14 | Comparative Example 40 | Example 15 | Comparative Example 41 |
|---|---|---|---|---|---|---|---|---|---|---|
| pH of process solution | 4 | 6 | 4 | 6 | 4 | 6 | 4 | 6 | 4 | 6 |
| Type of additive | EPI-AMINE | | polyDADMAC | | PAM | | PEI | | PVAM | |
| **Results** | | | | | | | | | | |
| Precipitate formation | O | X | O | X | O | X | O | X | O | X |
| Permeate flux (LMH) | 1938 | 804 | 1862 | 796 | 2775 | 651 | 1989 | 427 | 1769 | 826 |
| UV (280nm) | 16 | 24 | 16 | 21 | 14 | 20 | 14 | 20 | 13 | 23 |
| UV (420nm) | 1.1 | 4.6 | 0.7 | 4.0 | 0.8 | 1.0 | 1.8 | 3.9 | 2.0 | 4.4 |

As shown in Tables 2 to 7, in the samples without a coagulant/flocculant tested in the leucine production process, no precipitation was observed regardless of pH, and permeate flux was low. Among the groups treated with coagulants/flocculants, the group treated with APAM similarly showed no precipitation regardless of pH, and no improvement in permeate flux was observed compared to the control without a coagulant/flocculant. The group treated with the inorganic coagulant PAC showed precipitation at pH 4, but only a slight improvement in permeate flux was observed, and no precipitation was observed at the other pH values. Meanwhile, in the groups treated with EPI-AMINE, polyDADMAC, PAM, PEI, or PVAM, no precipitation and no improvement in permeate flux were observed in the test groups adjusted to pH 6, whereas precipitation and a significant increase in permeate flux were observed in the test groups adjusted to pH 4. In particular, the group treated with PAM was confirmed to have an improvement of about 4.0-fold in filtration flux. Moreover, these test groups exhibited a significant decrease in the absorbance at 420 nm, which is an index of chromaticity. Such results indicate that the quality of the filtrate can be improved by the above process.

Overall, the method of the present disclosure, which further comprises adding a coagulant/flocculant prior to membrane filtration, can significantly improve the average membrane permeate flux while maintaining the concentration ratio at an equivalent level compared to that of the case without a coagulant/flocculant, thereby markedly increasing the daily production of amino acids. Accordingly, it suggests that an improved process that offers superior cost competitiveness can be provided. Moreover, the method of the present disclosure was specifically confirmed to significantly reduce the absorbance at 420 nm, which is a product specification requirement in the food-grade amino acid industry, by efficiently removing impurities including colored substances as well as proteins, and thus can be useful for improving the quality of amino acid products.

As set forth above, those skilled in the art will be able to understand that the present disclosure may be embodied in other specific forms without departing from the technical spirit or essential characteristics thereof. In this regard, the embodiments disclosed herein are only for illustrative purposes and should not be construed as limiting the scope of the present disclosure. The scope of the present disclosure should be construed as including the meaning and scope of the appended claims rather than the detailed description, and all changes or variations derived from the equivalent concepts fall within the scope of the present disclosure.

**Claims**

1. A method for preparing an amino acid from a culture broth comprising an amino acid, comprising:

   (a) adding an organic coagulant, a cationic polymer flocculant, or both to the culture broth comprising an amino acid to precipitate impurities; and
   (b) subjecting the culture broth with precipitated impurities to membrane filtration.

2. The method according to claim 1,
   wherein the organic coagulant is poly(dimethylamine-co-epichlorohydrin) (EPI-AMINE) or poly(diallyldimethylammonium chloride) (polyDADMAC), and the cationic polymer flocculant is polyacrylamide (PAM), polyethyleneimine (PEI), or polyvinylamine (PVAM).

3. The method according to claim 1,
   further comprising adjusting the culture broth comprising an amino acid to an acidic pH of 3 to 5.

4. The method according to claim 1,
   wherein the filtration flux of the membrane filtration is improved.

5. The method according to claim 1,
   wherein the chromaticity of the membrane-filtered filtrate is improved and the content of impurities in the filtrate is reduced.

6. The method according to claim 1,
   wherein the impurities are one or more selected from the group consisting of cells, proteins, colloidal substances, natural organic matter (NOM), and low-molecular-weight substances.

7. The method according to claim 1,
   wherein the culture broth comprising an amino acid is obtained through fermentation by a microorganism.

8. The method according to claim 1,

further comprising a pretreatment step of separating cells from the culture broth comprising an amino acid.

9. The method according to claim 1,
   further comprising, after the membrane filtration step, optionally one or more steps selected from the group consisting of a decolorization step, a filtrate concentration step, a crystallization step, a crystal separation step, a drying step, a separation step, and a product commercialization step.

## INTERNATIONAL SEARCH REPORT

| | International application No. |
|---|---|
| | **PCT/KR2024/095489** |

### A. CLASSIFICATION OF SUBJECT MATTER

**C12P 13/00**(2006.01)i; **C12N 1/20**(2006.01)i; **B01D 61/00**(2006.01)i; **B01D 17/00**(2006.01)i

According to International Patent Classification (IPC) or to both national classification and IPC

### B. FIELDS SEARCHED

Minimum documentation searched (classification system followed by classification symbols)

C12P 13/00(2006.01); B01D 21/01(2006.01); C07C 227/40(2006.01); C07C 229/08(2006.01); C07C 67/60(2006.01); C07K 1/30(2006.01); C07K 14/745(2006.01); C12P 13/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 배양액(culture media), 발효(fermentation), 불순물(impurity), 응결제(coagulant), 응집제(flocculant), 아미노산(amino acid), 정제(purification)

### C. DOCUMENTS CONSIDERED TO BE RELEVANT

| Category* | Citation of document, with indication, where appropriate, of the relevant passages | Relevant to claim No. |
|---|---|---|
| X | CN 101798273 A (GUANGDONG ZHAOQING XINGHU BIOLOGICAL TECHNOLOGY CO., LTD.) 11 August 2010 (2010-08-11) See claim 1; and paragraphs [0002], [0005]-[0006], [0010] and [0012]-[0013]. | 1-9 |
| A | KR 10-2018-0061510 A (OCI COMPANY LTD.) 08 June 2018 (2018-06-08) See claims 1, 5, 8 and 9. | 1-9 |
| A | KR 10-1995-0014294 A (MIWON CO.) 15 June 1995 (1995-06-15) See claims 1-5. | 1-9 |
| A | JP 2023-506467 A (TAKEDA PHARMACEUTICAL CO., LTD.) 16 February 2023 (2023-02-16) See paragraphs [0178]-[0181]. | 1-9 |
| A | CN 106278919 A (SHANDONG FUFENG FERMENTATION CO., LTD.) 04 January 2017 (2017-01-04) See claims 1-3. | 1-9 |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **26 June 2024** | **01 July 2024** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2024/095489**

| Patent document cited in search report | | | Publication date (day/month/year) | Patent family member(s) | | | Publication date (day/month/year) |
|---|---|---|---|---|---|---|---|
| CN | 101798273 | A | 11 August 2010 | CN | 101798273 | B | 01 May 2013 |
| KR | 10-2018-0061510 | A | 08 June 2018 | | None | | |
| KR | 10-1995-0014294 | A | 15 June 1995 | | None | | |
| JP | 2023-506467 | A | 16 February 2023 | AR | 120710 | A1 | 09 March 2022 |
| | | | | CN | 115380044 | A | 22 November 2022 |
| | | | | EP | 4073104 | A2 | 19 October 2022 |
| | | | | TW | 202136283 | A | 01 October 2021 |
| | | | | US | 2023-017907 | A1 | 19 January 2023 |
| | | | | WO | 2021-116273 | A2 | 17 June 2021 |
| | | | | WO | 2021-116273 | A3 | 05 August 2021 |
| CN | 106278919 | A | 04 January 2017 | CN | 106278919 | B | 16 January 2018 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8465962 B2
- US 9885093 B2
- US 10351859 B2
- US 7863435 B2
- US 10787692 B2
- US 9029105 B2
- US 20210094903 A1
- US 9587261 B2